# EUROPEAN PATENT SPECIFICATION

(11) **EP 3 131 501 B1**
(45) Date of publication and mention of the grant of the patent: **07.06.2023**
(21) Application number: 14727615.8
(22) Date of filing: 18.04.2014
(51) Int. Cl.: A61F 2/16

(54) **SECONDARY INTRAOCULAR LENS WITH MAGNIFYING COAXIAL OPTICAL PORTION**
SEKUNDÄRE INTRAOKULARLINSE MIT VERGRÖSSERNDEM KOAXIALEN OPTISCHEN ABSCHNITT
LENTILLE INTRAOCULAIRE SECONDAIRE AYANT UNE PARTIE OPTIQUE COAXIALE GROSSISSANTE

(43) Date of publication of application: 22.02.2017
(73) Proprietor: Medicontur Holding Ltd., 2072 Zsámbék (HU)
(72) Inventor: SCHARIOTH, Gábor B., 45657 Recklinghausen (DE); KONTUR, Laszlo F., H-1123 Budapest (HU)
(74) Representative: Kacsuk, Zsófia
(86) International application number: PCT/HU2014/000032
(87) International publication number: WO 2015/159111

(56) References cited:
- EP-A1- 1 818 023
- US-A1- 2005 027 355
- US-A1- 2005 288 784
- US-A1- 2006 206 206
- US-A1- 2006 259 138
- US-A1- 2007 270 947

## Description

### TECHNICAL FIELD

The present invention relates to a secondary (so-called Add-on) intraocular lens, IOL, for surgical implantation in a patient's pseudo-phakic eye, i.e. in addition to at least one primary IOL that has already been implanted in the posterior chamber of the patient's eye prior to the implantation of said secondary IOL.

### BACKGROUND

Age-related Macular degeneration (AMD) is a medical condition that affects the center of retina (macula) in elderly patients and is leading to loss of central vision. Peripheral visual field is usually not affected and patients keep ability for orientation. Nontheless most patients loose at least in the late stage of the disease the ability to read and AMD is the leading cause for blindness and visual impairment in patients older then 50 years in the western world.

Numerous surgical interventions with implantation of special lenses and devices have been proposed. Some systems base on magnification of the image, but at the same time causing severe reduction of the visual field, like some implantable telescope, as described in EP1475055. This solution did not become popular because of the reduction of the visual field and because it is bulky and difficult to implant, further it is contraindicated in single eyed patients.

Other systems using Fresnel Lens systems, like described in patent application WO2005039451 or combined converging and diverging lenses with non-coincident axes, like described in patent applications WO2010136798 and WO2010131955, proposed to optically divert the light beam and displace the focus to an area of the retina outside the fovea. These systems became not popular because displacing the focus to other areas of the retina than the fovea will not allow reading vision as these parts of the retina have a reduced intensity of photoreceptor cells.

Other systems proposed the combination of special intraocular implants having at least one negative intraocular lens portion interacting with an external lens (spectacles), like described in applications WO0132105 and EP2319457. These systems did not become popular because the use of special spectacles is required, therefore they do not offer any advantage over classical magnification glasses.

Patent applications WO8707496 - and WO8909576 respective - describe a one-piece bifocal intraocular lens construction in a coaxial embodiment, mentioning the constriction of the pupil. However, the lens in the applications is described as a stand-alone lens, not designed as a secondary IOL that has to optically co-operate with a primary IOL. Further aforementioned lens is described as a rigid lens only. Finally the power distribution of the lens in the aforementioned documents is limited to the use for presbyopia claiming addition of 2 - 4 D in the central lens portion for near vision.

The main problem with all aforementioned inventions is that the systems proposed are usually designed to be implanted instead of a standard intraocular lens and most surgeons would object to that choice.

As a matter of fact, more than two thirds of patients with advanced AMD and visual acuity of 0.3 or less are pseudo-phakic already, i.e. they have had cataract surgery with implantation of an intraocular lens into the capsular bag.

### SUMMARY

With this invention we have set ourselves the objective to deliver a simple, cheap and safe solution for improving the near vision of pseudo-phakic patients suffering from Age-related Macular Degeneration (AMD).

We realized that we can benefit from the effect of near vision miosis in which the pupil constricts in a reflex when the eye focuses on a near object. This reflex also works reliably in elderly people. The constriction of the pupil limits the light beam to the center of the lens in the eye.

Our invention is designed for pseudo-phakic patients suffering from AMD by using miosis as one of the three natural eye reflexes being part of the so-called Near Triad, i.e. the decrease in size of the pupil that accompanies accommodation and convergence of the two eyes.

Accordingly, the invention relates to a secondary (so-called Add-on) intraocular lens, IOL, that is made from a foldable soft material like acrylate or silicone. The secondary IOL is adapted to be surgically implanted into the sulcus ciliaris of a patient's pseudo-phakic eye, i.e. in addition to at least one primary IOL that has already been implanted in the posterior chamber of the patient's eye prior to the implantation of said secondary IOL.

The secondary IOL is designed to optically co-operate with the primary IOL in order to coaxially focus a combined image on the retina of the patient's eye improving the visual capabilities of the patient by additionally magnifying at least a central part of the image of the primary IOL projected onto the fovea of the retina in order to enhance near vision.

The foldable secondary IOL comprises one or more haptics for fixing and stabilizing it within the sulcus ciliaris of the patient's eye - and an optically active portion designed to project the image through the primary IOL onto the retina.

The optically active portion of the secondary IOL comprises a central optical portion, preferably having a diameter smaller than 1.8 mm, and a peripheral optical portion extending around the central optical portion, thus forming two different, but coaxially positioned lenses from one block.

The central optical portion is designed to form a positive lens providing additional refractive power of preferably more than 5 diopters to the refractive power provided by the peripheral optical portion of the secondary IOL, thus providing additional refractive power of more than 5 diopters compared to the combined refractive power of the primary IOL and the peripheral optical portion of the secondary IOL.

With this construction, the patient is provided with the ability to have a magnified image without using spectacles or magnifying glass. If the patient is not satisfied with the secondary IOL, it can be removed surgically while keeping the function of the primary IOL. Due to the effect of near vision miosis, the central optical portion - providing the magnified image - will perform when the patient focuses on near objects only but will not influence significantly the far vision when patient focuses on distant objects through a dilated pupil.

Our invention is not intended and will not work for presbyopic, phakic or cataract patients. It targets solely pseudo-phakic patients with advanced AMD, offering them a convenient, simple and safe solution to restore their near vision impaired by AMD. In conclusion our invention can not be considered to be an improvement over aforementioned prior art - it is clearly different regarding purpose and function.

### BRIEF DESCRIPTION OF THE DRAWINGS

For a more complete understanding of the invention, reference is made to the following detailed description of an embodiment taken in conjunction with the accompanying drawings wherein:
FIGURE 1 shows the secondary IOL in side view;
FIGURE 2 depicts the secondary IOL in front view;
FIGURE 3 shows the patient's eye with constricted pupil;
FIGURE 4 shows the patient's eye with distended pupil.

### DETAILED DESCRIPTION

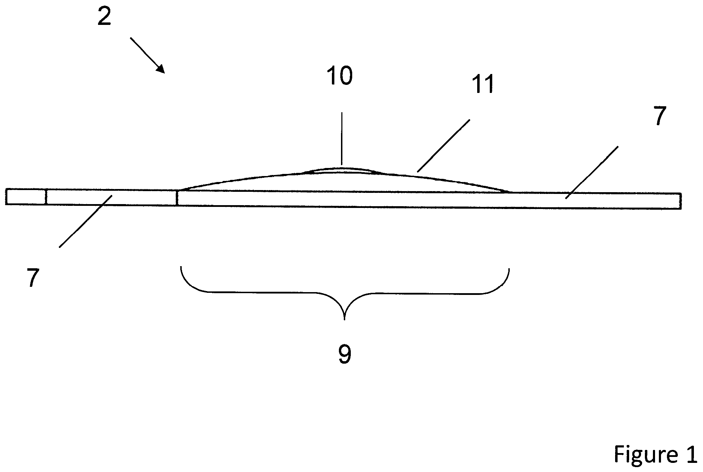

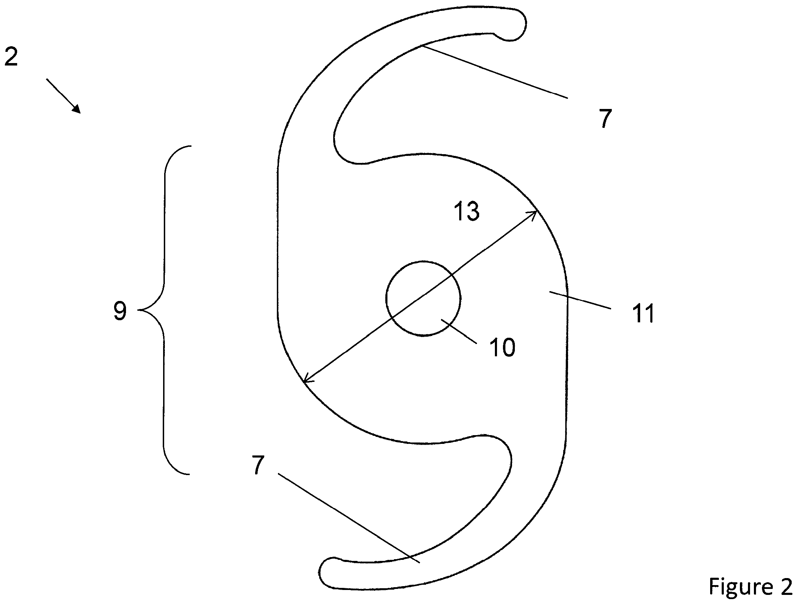

As it is described in Figures 1 - 2, a secondary intraocular lens, IOL, is provided made from foldable soft material like acrylate or silicone. The secondary IOL 2 comprises haptics 7 for fixing and stabilizing it within the patient's eye and an optically active portion 9. These haptics can be of any shape that is known in current IOLs: open C-shaped (as in our illustration) or Z-shaped loop, closed loops or plate haptics, with or without fenestration, with or without axial angulation.

The optically active portion 9 comprises a central optical portion 10 and a peripheral optical portion 11 extending around the central optical portion 10. The central optical portion 10 and a peripheral optical portion 11 form two different, but coaxially positioned lenses from one block. The optically active portion 9 may have a diameter 13 between 4 mm and 10 mm, preferably between 5 mm and 7 mm.

The central optical portion 10 is designed to form a positive lens providing additional refractive power to the refractive power provided by the peripheral optical portion 11 of the secondary IOL 2.

The diameter of the central optical portion 10 may be smaller than 1.8 mm in order to fully use but not to exceed the diameter of the constricted pupil (by much) and not to disturb far vision through the dilated pupil in a significant way. The central optical portion 10 may have a diameter of bigger than 0.5 mm in order to produce the minimal desired magnifying effect that can be perceived by the patient. Preferably the central optical portion 10 may have a diameter between 0.8 mm and 1.6 mm in order to produce a sound balance between the above mentioned conditions.

The additional refractive power of the central optical portion 10 over the peripheral optical portion 11 may be more than 5 diopters in order to produce a magnification that could restore the patient's reading capability. The additional refractive power of the central optical portion 10 over the peripheral optical portion 11 may be less than 25 diopters because in real life it would be hard to handle any object closer to the eye than 4 cm. Therefore the central optical portion 10 may have a refractive power of between +5 diopters and +25 diopters, preferably between +8 diopters and + 12 diopters in addition to the peripheral optical portion 11. Thus this invention can provide additional refractive power of between +5 diopters and +25 diopters, preferably between +8 diopters and + 12 diopters compared to the combined refractive power of a primary IOL 1 and the peripheral optical portion 11 of the secondary IOL 2.

The peripheral optical portion 11 may be designed to form a lens with zero refractive power, thus not interfering with the image provided by the primary IOL 1, leaving the patient most of his vision provided by the primary IOL 1. In another embodiment the peripheral optical portion 11 may be designed to form a lens with a certain refractive power between -5D and + 15D in order to correct any prior error in refraction or any unintended, undesired change in the patient's vision provided by the primary IOL 1.

The ratio between the diameters of the central optical portion 10 and the optically active portion 9 of the secondary IOL 2 may be between 0.05 and 0.45, preferable between 0.15 and 0.35.

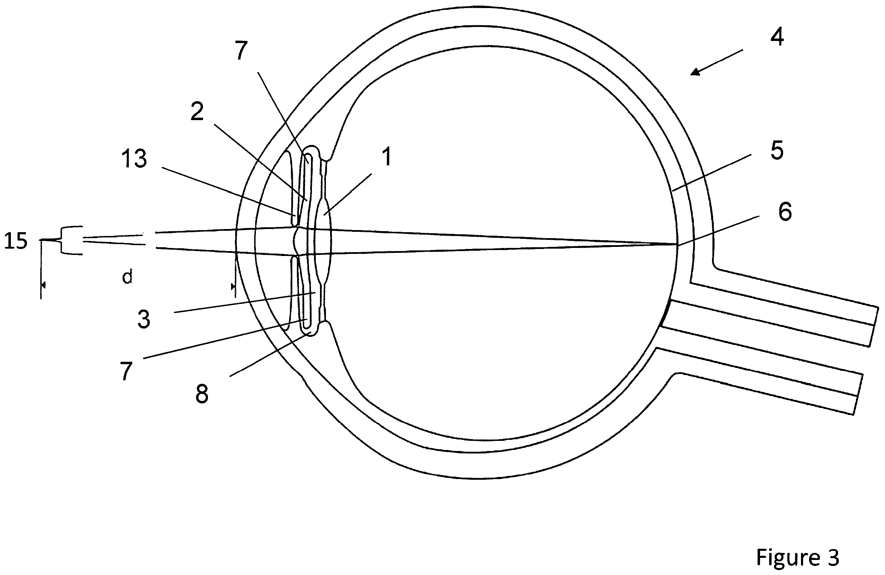

In Figures 3-4, the position of the secondary IOL 2 is depicted in the patient's eye 4. As it is seen, the secondary IOL 2 has been surgically implanted in a pseudo-phakic eye, i.e. in addition to at least one primary IOL 1 that has already been implanted in the posterior chamber 3 of the patient's eye 4 prior to the implantation of said secondary IOL 2. The haptics 7 fix and stabilize the secondary IOL 2 within the sulcus ciliaris 8 of the patient's eye 4. The optically active portion 9 is designed to project the image through the primary IOL 1 onto the retina 5. The secondary IOL 2 is arranged optically coaxial to the primary IOL 1 focusing a combined image on the retina 5 additionally magnifying at least a central part of the image of the primary IOL 1 projected onto the macula 6 of the retina 5. In this way, the secondary IOL 2 improves the visual capabilities of the patient by additionally magnifying at least a central part of the image of the primary IOL 1.

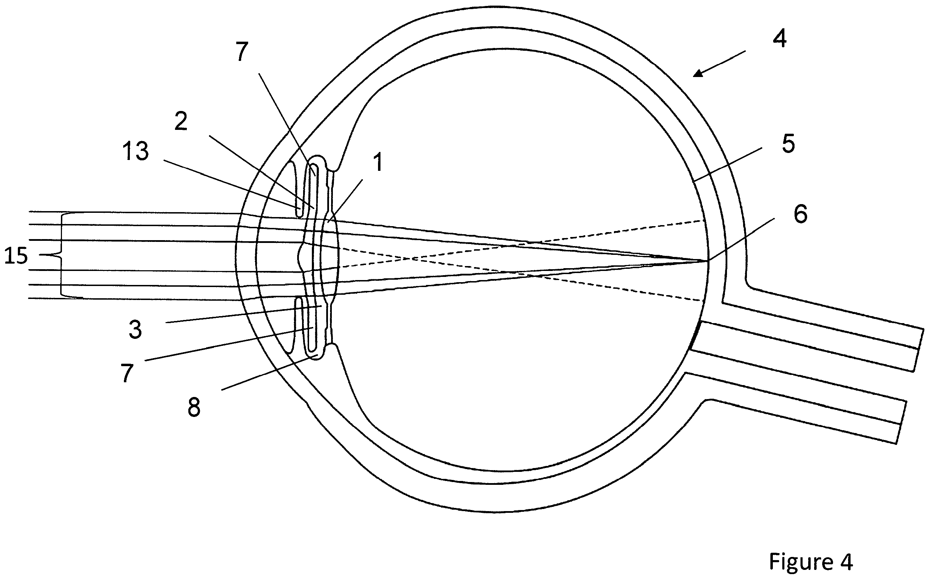

The effect of the secondary IOL 2 can be understood by comparing Figure 3 and Figure 4. Figure 3 and Figure 4 differ in the size of the pupil 15 formed by the iris 13.

In Figure 3, the pupil 15 is constricted, so the light beam is restricted mainly to the central optical portion 10 of the secondary IOL 2 providing a magnified image on the macula 6. This is the case when the patient focuses on nearby objects, i.e. reading a newspaper or a price tag, and the reflex of near vision miosis constricts the pupil 15. The image thus projected onto the retina 5 is magnified compared to the image produced by distant vision in the eye, which enables the patient's eye to resolve the image in case of AMD as well. Due to the relatively high refractive power of the central optical portion 10 comparing to the basic lens power, the sharp vision is at a very near distance, d, for which the typical value is 5-20 cm.

In Figure 4, the patient focuses on a distant object and the pupil 15 is dilated leaving enough space around the central optical portion 10 for the light rays passing through the peripheral optical portion 11 of the secondary IOL 2 as well. Thus the light rays coming from a distant object passing the peripheral optical portion 11 and forming the targeted distant image on the retina will dominate in the patient's perception over the rays passing the central optical portion 10 that do not focus onto the retina (dashed lines).

Although one preferred embodiment of the present invention has been illustrated in the accompanying drawings and described in the foregoing detailed description, it is understood that the invention is not limited to the disclosed embodiment but is capable of numerous rearrangements, modifications, and substitutions for IOL injectors without departing from the invention.

## Claims

1. A secondary intraocular lens, IOL, (2) for improving the near vision of pseudo-phakic patients suffering from age-related macular degeneration, made from a foldable soft material, for surgical implantation in a sulcus ciliaris of a patient's eye (4) in addition to at least one primary IOL (1) that has already been implanted in the posterior chamber (3) of the patient's eye (4) prior to the implantation of said secondary IOL (2); wherein
- the secondary IOL (2) comprises one or more haptics (7) for fixing and stabilizing it within the sulcus ciliaris (8) of the patient's eye (4) - and an optically active portion (9) designed to project the image through the primary IOL (1) onto the retina (5);
- the optically active portion (9) of the secondary IOL (2) comprises a central optical portion (10) and a peripheral optical portion (11) extending around the central optical portion (10), forming two different, but coaxially positioned lenses from one block;
- the peripheral optical portion (11) providing a first refractive power and the central optical portion (10) is designed to form a positive lens providing additional refractive power over the first refractive power provided by the peripheral optical portion (11) of the secondary IOL (2);
- the secondary IOL (2) is configured to be arranged optically coaxial to the primary IOL (1) for focusing a combined image on the retina (5) of the patient's eye (4) additionally magnifying at least a central part of the image of the primary IOL (1) projected onto the macula (6) of the retina (5).

2. The secondary IOL (2) as claimed in claim 1, wherein the diameter of the central optical portion (10) is smaller than 1.8 mm.

3. The secondary IOL (2) as claimed in claim 1, wherein the additional refractive power of the central optical portion (10) is more than 5 diopters.

4. The secondary IOL (2) as claimed in claim 1, wherein the peripheral optical portion (11) is designed to form a lens with zero refractive power, thus not interfering with the image provided by the primary IOL (1).

5. The secondary IOL (2) as claimed in claim 1, wherein the peripheral optical portion (11) is designed to form a lens with a certain refractive power between -5D and + 15D designed to correct any prior error in refraction or any unintended, non-optimal change in the patient's vision provided by the primary IOL (1).

6. The secondary IOL (2) as claimed in claim 1, wherein the central optical portion (10) of the secondary IOL (2) has a refractive power of between +5 diopters and +25 diopters, preferably between +8 diopters and + 12 diopters in addition to the peripheral optical portion (11) of the secondary IOL (2), providing additional refractive power of between +5 diopters and +25 diopters, preferably between +8 diopters and + 12 diopters compared to the combined refractive power of the primary IOL (1) and the peripheral optical portion (11) of the secondary IOL (2).

7. The secondary IOL (2) as claimed in claim 1, wherein the optically active portion (9) has a diameter (13) between 4 and 10 mm, preferably between 5 and 7 mm.

8. The secondary IOL (2) as claimed in claim 1, wherein the central optical portion (10) has a diameter of bigger than 0.5 mm and smaller than 1.8 mm, preferably between 0.8 and 1.6 mm.

9. The secondary IOL (2) as claimed in claim 1, wherein the ratio between the diameter of the central optical portion (10) and the optically active portion (9) of the secondary IOL (2) is between 0.05 and 0.45, preferable between 0.15 and 0.35.

## Patentansprüche

1. Sekundäre Intraokularlinse, IOL, (2) zur Verbesserung der Nahsicht pseudophakischer Patienten, die an altersbedingter Makuladegeneration leiden, hergestellt aus einem faltbaren weichen Material, zur chirurgischen Implantierung in einen Sulcus ciliaris eines Auges eines Patienten (4) zusätzlich zu mindestens einer primären IOL (1), die bereits vor der Implantierung der sekundären IOL (2) in der hinteren Kammer (3) des Auges des Patienten (4) implantiert worden ist; wobei
- die sekundäre IOL (2) eine oder mehrere Haptiken (7) zu ihrer Befestigung und Stabilisierung innerhalb des Sulcus ciliaris (8) des Auges des Patienten (4) und einen optisch aktiven Teil (9), der ausgebildet ist, um das Bild durch die primäre IOL (1) auf die Netzhaut (5) zu projizieren, umfasst;
- der optisch aktive Teil (9) der sekundären IOL (2) einen zentralen optischen Teil (10) und einen peripheren optischen Teil (11), der sich um den zentralen optischen Teil (10) herum erstreckt, umfasst und zwei unterschiedliche, aber koaxial positionierte Linsen aus einem Block bildet;
- der periphere optische Teil (11) eine erste Brechkraft bereitstellt und der zentrale optische Teil (10) ausgebildet ist, um eine positive Linse zu bilden, die eine zusätzliche Brechkraft zur vom peripheren optischen Teil (11) der sekundären IOL (2) bereitgestellten ersten Brechkraft bereitstellt, sodass im zentralen optischen Teil (10) der sekundären IOL (2) im Vergleich zur vom peripheren optischen Teil (11) bereitgestellten ersten Brechkraft eine höhere Brechkraft erzielt wird;
- die sekundäre IOL (2) konfiguriert ist, um zum Richten eines kombinierten Bildes auf die Netzhaut (5) des Auges des Patienten (4) optisch koaxial zur primären IOL (1) angeordnet zu sein, wodurch mindestens ein zentraler Teil des Bildes der primären IOL (1), das auf die Makula (6) der Netzhaut (5) projiziert wird, zusätzlich vergrößert wird.

2. Sekundäre IOL (2) nach Anspruch 1, wobei der Durchmesser des zentralen optischen Teils (10) kleiner als 1,8 mm ist.

3. Sekundäre IOL (2) nach Anspruch 1, wobei die zusätzliche Brechkraft des zentralen optischen Teils (10) mehr als 5 Dioptrien beträgt.

4. Sekundäre IOL (2) nach Anspruch 1, wobei der periphere optische Teil (11) ausgebildet ist, um eine Linse mit null Brechkraft zu bilden, und somit das von der primären IOL (1) bereitgestellte Bild nicht stört.

5. Sekundäre IOL (2) nach Anspruch 1, wobei der periphere optische Teil (11) ausgebildet ist, um eine Linse mit einer bestimmten Brechkraft zwischen -5 D und +15 D zu bilden, die ausgebildet ist, um frühere Brechungsfehler oder unbeabsichtigte, nicht optimale Veränderungen der Sehkraft des Patienten, bereitgestellt von der primären IOL (1), zu korrigieren.

6. Sekundäre IOL (2) nach Anspruch 1, wobei der zentrale optische Teil (10) der sekundären IOL (2) eine Brechkraft zwischen +5 Dioptrien und +25 Dioptrien, vorzugsweise zwischen +8 Dioptrien und +12 Dioptrien, zusätzlich zum peripheren optischen Teil (11) der sekundären IOL (2) aufweist, wobei im Vergleich zur kombinierten Brechkraft der primären IOL (1) und des peripheren optischen Teils (11) der sekundären IOL (2) eine zusätzliche Brechkraft zwischen +5 Dioptrien und +25 Dioptrien, vorzugsweise zwischen +8 Dioptrien und +12 Dioptrien, bereitstellt wird.

7. Sekundäre IOL (2) nach Anspruch 1, wobei der optisch aktive Teil (9) einen Durchmesser (13) zwischen 4 und 10 mm, vorzugsweise zwischen 5 und 7 mm, aufweist.

8. Sekundäre IOL (2) nach Anspruch 1, wobei der zentrale optische Teil (10) einen Durchmesser von größer als 0,5 mm und kleiner als 1,8 mm, vorzugsweise zwischen 0,8 und 1,6 mm, aufweist.

9. Sekundäre IOL (2) nach Anspruch 1, wobei das Verhältnis zwischen dem Durchmesser des zentralen optischen Teils (10) und des optisch aktiven Teils (9) der sekundären IOL (2) zwischen 0,05 und 0,45, vorzugsweise zwischen 0,15 und 0,35, liegt.

## Revendications

1. Lentille intraoculaire secondaire, IOL, (2) pour améliorer la vision de près de patients pseudophaques souffrant de dégénérescence maculaire liée à l'âge, réalisée à partir d'un matériau souple pliable, pour une implantation chirurgicale dans un sulcus ciliaire d'un oeil d'un patient (4) en plus d'au moins une IOL principale (1) qui a déjà été implantée dans la chambre postérieure (3) de l'œil du patient (4) avant l'implantation de ladite IOL secondaire (2) ; dans laquelle
- l'IOL secondaire (2) comprend une ou plusieurs haptiques (7) pour la fixer et la stabiliser au sein du sulcus ciliaire (8) de l'œil du patient (4) - et une portion optiquement active (9) conçue pour projeter l'image à travers l'IOL principale (1) sur la rétine (5) ;
- la portion optiquement active (9) de l'IOL secondaire (2) comprend une portion optique centrale (10) et une portion optique périphérique (11) s'étendant autour de la portion optique centrale (10), formant deux lentilles différentes à partir d'un bloc mais positionnées coaxialement ;
- la portion optique périphérique (11) fournissant une première puissance optique et la portion optique centrale (10) est conçue pour former une lentille positive fournissant une puissance optique additionnelle à la première puissance optique fournie par la portion optique périphérique (11) de l'IOL secondaire (2), obtenant ainsi une puissance optique plus forte dans la portion optique centrale (10) de l'IOL secondaire (2) par rapport à la première puissance optique fournie par la portion optique périphérique (11) ;
- l'IOL secondaire (2) est configurée pour être agencée optiquement coaxiale à l'IOL principale (1) pour focaliser une image combinée sur la rétine (5) de l'œil du patient (4) grossissant additionnellement au moins une partie centrale de l'image de l'IOL principale (1) projetée sur la macula (6) de la rétine (5).

2. IOL secondaire (2) selon la revendication 1, dans laquelle le diamètre de la portion optique centrale (10) est inférieur à 1,8 mm.

3. IOL secondaire (2) selon la revendication 1, dans laquelle la puissance optique additionnelle de la portion optique centrale (10) est supérieure à 5 dioptres.

4. IOL secondaire (2) selon la revendication 1, dans laquelle la portion optique périphérique (11) est conçue pour former une lentille avec une puissance optique égale à zéro, n'interférant ainsi pas avec l'image fournie par l'IOL principale (1).

5. IOL secondaire (2) selon la revendication 1, dans laquelle la portion optique périphérique (11) est conçue pour former une lentille avec une certaine puissance optique entre -5D et +15D conçue pour corriger une quelconque erreur antérieure de refraction ou un quelconque changement involontaire non optimal de la vision du patient fournie par l'IOL principale (1).

6. IOL secondaire (2) selon la revendication 1, dans laquelle la portion optique centrale (10) de l'IOL secondaire (2) présente une puissance optique entre +5 dioptres et +25 dioptres, de préférence entre +8 dioptres et +12 dioptres en plus de la portion optique périphérique (11) de l'IOL secondaire (2), fournissant une puissance optique additionnelle entre +5 dioptres et +25 dioptres, de préférence entre +8 dioptres et +12 dioptres par rapport à la puissance optique combinée de l'IOL principale (1) et de la portion optique périphérique (11) de l'IOL secondaire (2).

7. IOL secondaire (2) selon la revendication 1, dans laquelle la portion optiquement active (9) présente un diamètre (13) entre 4 et 10 mm, de préférence entre 5 et 7 mm.

8. IOL secondaire (2) selon la revendication 1, dans laquelle la portion optique centrale (10) présente un diamètre supérieur à 0,5 mm et inférieur à 1,8 mm, de préférence entre 0,8 et 1,6 mm.

9. IOL secondaire (2) selon la revendication 1, dans laquelle le rapport entre le diamètre de la portion optique centrale (10) et la portion optiquement active (9) de l'IOL secondaire (2) se situe entre 0,05 et 0,45, de préférence entre 0,15 et 0,35.
